## Europäisches Patentamt
## European Patent Office
## Office européen des brevets

(11) Veröffentlichungsnummer: **0 027 662**
A1

(12) # EUROPÄISCHE PATENTANMELDUNG

(21) Anmeldenummer: 80106503.8

(22) Anmeldetag: 23.10.80

(51) Int. Cl.³: **C 12 N 15/00**
A 61 K 31/70, A 61 K 39/00
A 61 K 9/50, A 01 K 67/00
A 01 H 1/00, C 12 N 5/00
C 12 N 1/16

(30) Priorität: 23.10.79 DE 2942780

(43) Veröffentlichungstag der Anmeldung:
29.04.81 Patentblatt 81/17

(84) Benannte Vertragsstaaten:
BE CH DE FR GB IT LI NL SE

(71) Anmelder: Max-Planck-Gesellschaft zur Förderung der Wissenschaften e.V.
Bunsenstrasse 10
D-3400 Göttingen(DE)

(72) Erfinder: Hofschneider, Peter Hans
Bavariaring 12
D-8000 München 2(DE)

(72) Erfinder: Wong, Tai-Kin
Guardinistrasse 44
D-8000 München 70(DE)

(72) Erfinder: Nicolau, Yves-Claude
Leonhard-Stinnes-Strasse 48
D-4330 Mülheim Ruhr(DE)

(74) Vertreter: Rauh, Peter A., Dr.
Siebertstrasse 4 Postfach 86 07 67
D-8000 München 86(DE)

(54) Eukaryotische Zellen, eukaryotische Protoplasten und vielzellige eukaryotische lebende Organismen mit einem Gehalt an durch Lipidvesikel eingebrachter DNA, Verfahren zu ihrer Herstellung und ihre Verwendung zur Herstellung von Gen-Produkten, zur Immunisierung und zur Behebung genetisch bedingter Defekte.

(57) Eukaryotische Zellen, eukaryotische Protoplasten und vielzellige eukaryotische lebende Organismen mit einem Gehalt an durch Lipidvesikel eingebrachter DNA, Verfahren zu ihrer Herstellung und ihre Verwendung zur Herstellung von Gen-Produkten, zur Immunisierung und zur Behebung genetisch bedingter Defekte.

EP 0 027 662 A1

PATENTANWÄLTE

**0027662**

SIEBERTSTRASSE 4 · 8000 MÜNCHEN 86 · PHONE: (089) 47 40 75
CABLE: BENZOLPATENT MÜNCHEN · TELEX 5-29 453 VOPAT D

u.Z.: P 849 EP (Vo/Ra/ger)

MAX-PLANCK-GESELLSCHAFT

zur Förderung der Wissenschaften e.V.

3400 Göttingen, Bunsenstraße 10

" Eukaryotische Zellen, eukaryotische Protoplasten und vielzellige eukaryotische lebende Organismen mit einem Gehalt an durch Lipidvesikel eingebrachter DNA, Verfahren zu ihrer Herstellung und ihre Verwendung zur Herstellung von Gen-Produkten, zur Immunisierung und zur Behebung genetisch bedingter Defekte "

Priorität: 23. Oktober 1979, Bundesrepublik Deutschland, Nr. P 29 42 780.3

Die Erfindung betrifft eukaryotische Zellen, eukaryotische Protoplasten und vielzellige eukaryotische lebende Organismen mit einem Gehalt an durch Lipidvesikel eingebrachter DNA, die die genetische Information in Form nützlicher Gen-Produkte exprimieren können, Verfahren zu ihrer Herstellung und ihre Verwendung zur Herstellung von Gen-Produkten, zur Immunisierung und zur Behebung genetisch bedingter Defekte.

Lipidvesikel stellen annähernd kugelförmige Gebilde dar, deren Wandung aus einem System von mindestens einer Membran aus Lipidmolekülen besteht. Zur Zeit sind drei verschiedene Arten von Lipidvesikeln bekannt, nämlich multilamellare Lipidvesikel (MLV), auch als Liposomen bezeichnet, kleine unilamellare Lipidvesikel (SUV) sowie große unilamellare Lipidvesikel (LUV). Die Herstellung, die Eigenschaften und die Verwendung dieser Lipidvesikel ist u.a. von G. Poste,

D. Papahadjopoulos und W.J. Vail, Lipid Vesicles as Carriers for Introducing Biologically Active Materials into Cells, Methods in Cell Biology, Bd. 14 (1976), S. 33 - 71, sowie R.E.Pagano und J.N.Weinstein, Ann. Rev. Biophys. Bioeng., Bd. 7 (1978), S. 435 bis 468, beschrieben. In diesen beiden Übersichtsartikeln ist auf umfangreiche weitere Literatur verwiesen worden.

Es ist bekannt, daß Warmblüterzellen, z.B. Säugetierzellen, in vitro und in vivo eine große Anzahl von Lipidvesikeln ohne cytotoxische Wirkungen aufnehmen. Da die verschiedensten Stoffe im Inneren von Lipidvesikeln oder zwischen den Vesikelmembranen eingeschlossen werden können, kann die Aufnahme von Lipidvesikeln bestimmter Zusammensetzung durch Zellen eine Möglichkeit zur Modifizierung der zellulären Zusammensetzung und zum Einführen nicht-permeabler biologisch aktiver Stoffe in die Zelle eröffnen; vgl. G. Poste, D. Papahadjopoulos und W.J. Vail, a.a.O. und G. Gregoriadis, The Carrier Potential of Liposomes in Biology and Medicine, The New England Journal of Medicine, Bd. 295 (1976), S. 704 bis 710, und R.E. Pagano und J.N. Weinstein, a.a.O.

So ist z.B. bekannt, Inositolhexaphosphat enthaltende unilamellare Lipidvesikel in das Zellinnere von Erythrocyten einzubringen; vgl. K. Gersonde und C. Nicolau, Blut, Bd. 39 (1979), S. 1 bis 7. Ferner ist bekannt, daß Metaphase-Chromosomen, die das HGPRT-Gen bzw. das AMP-Pyrophosphat-Phosphoribosyltransferase-Gen (aprt[+]) tragen, mit Liposomen in Zellen überführt werden können, die HGPRT bzw. aprt-negativ sind. Die Überführung läßt sich durch Selektionierung der wenigen HGPRT bzw. aprt-positiv gewonnenen Zellen nachweisen; vgl. A.B. Mukherjee u. Mitarb., Proc. Natl. Acad. Sci. USA, Bd. 75 (1978), S. 1361 - 1365, und M. Wigler u. Mitarb., Proc. Natl. Acad. Sci. USA, Bd. 76 (1979) S. 1373 bis 1376. Es ist auch bekannt, daß Polioviren Typ I bzw. die replikationsfähige RNA dieser Viren mit Hilfe von Lipidvesikeln in Poliovirus-resistente Ovarienzellen des chinesischen Hamsters überführt werden. In diesem Fall wird die Überführung durch Nachweis der Virusproduktion erbracht; vgl. T. Wilson u. Mitarb., Proc. Natl. Acad. Sci. USA, Bd. 74 (1977), S. 3471 bis 3475, und T. Wilson u. Mitarb., Cell, Bd. 17 (1979), S. 77 bis 84.

Auch die Boten-RNA (m-RNA) für Kaninchenglobin ist mit Lipidvesikeln (LUV) in Mäuse-Milzlymphocyten überführt worden, in denen dann die Synthese von Kaninchenglobin zu beobachten ist; vgl. G.J. Dimitriadis, Nature, Bd. 274 (1978), S. 923 bis 924. Die Verwendung von RNA hat allerdings den Nachteil, daß sie schwer zu reinigen ist und in gereinigtem Zustand sehr viel leichter der Hydrolyse unterliegt als DNA.

Das bakterielle selbst replikationsfähige Plasmid pBR322 kann mit Hilfe von Liposomen in E. coli eingeführt werden. Es repliziert sich in E. coli und kann durch die Nachkommenbakterien des Empfängerbakteriums, die nun die Resistenzmarker des pBR322 Plasmids zeigen, nachgewiesen werden; vgl. R.T. Fraley u. Mitarb., Proc. Natl. Acad. Sci. USA, Bd. 76 (1979), S. 3348 bis 3352. DNA-haltige Liposomen sind in den Kern der Protoplasten der Pflanze Vigna sinensis eingeführt worden; vgl. P.F. Lurquin, Nucleic Acids Research, Bd. 6 (1979), S. 3773 bis 3784. Es ist auch bekannt, daß Liposomen durch Injektion im lebenden Organismus zur Fusion mit Zellen dieses Organismus gebracht werden können.

Der Einschluß von Plasmid-DNA (pMB9) in Lipidvesikel (LUV) ist ebenfalls bekannt; vgl. G.J.Dimitriadis, Nucleic Acids Research, Bd. 6 (1979), S. 2697 bis 2705.

Der Erfindung liegt die Aufgabe zugrunde, eukaryotische Zellen, eukaryotische Protoplasten und vielzellige eukaryotische lebende Organismen mit einem Gehalt an durch Lipidvesikel eingebrachter einsträngiger oder doppelsträngiger DNA von Prokaryonten oder Eukaryonten zur Verfügung zu stellen mit dem Ziel der unmittelbaren Gen-Expression, d.h. der Synthese von nützlichen Gen-Produkten in den Zellen, und zwar in einer Menge, die deren einfache Erkennung, Anreicherung und Isolierung ermöglicht. Im Unterschied zu bisherigen Verfahren der Gen-Überführung (Gen-Transformation) soll es erfindungsgemäß möglich sein, die Gen-Produkte nach bekannten Methoden festzustellen und zu gewinnen, ohne daß die Zellen, welche die DNA-haltigen Lipidvesikel erhalten haben, von den übrigen Zellen selektioniert werden müssen. Außer den primären wertvollen Gen-Produkten (Proteine in Form von Enzymen, Virusantigene, Peptidhormone, biologisch aktive Polypeptide usw.) sollen auch sekundäre Gen-Produkte gewonnen werden können, die durch die enzymatische Leistung der primären Gen-Produkte, wie z.B. Enzyme, synthetisiert werden.

Eine weitere Aufgabe der Erfindung ist es, eukaryotische Zell-Linien zur Verfügung zu stellen, die nicht nur unmittelbar, sondern durch stabile Inkorporation der mittels Lipidvesikel eingeführten DNA auf die Dauer in Form ihrer Nachkommenzellen bestimmte wertvolle Gen-Produkte synthetisieren.

Eine weitere Aufgabe der Erfindung ist es, durch Einführung von DNA mittels Lipidvesikeln in vielzellige eukaryotische lebende Organismen ohne deren Integrität durch größere Eingriffe zu gefährden, die Produktion bestimmter Gen-Produkte zu veranlassen, z.B. die Bildung von Antigenen zwecks "Selbstimmunisierung", die Bildung von Antikörpern, oder die Bildung von Gen-Produkten zur zeitweiligen oder bleibenden Behebung genetisch bedingter Mängel, Defekte oder Krankheiten.

Eine weitere Aufgabe der Erfindung ist es, DNA enthaltende Lipidvesikel in das Cytoplasma von eukaryotischen Protoplasten aus Pflanzen einzuführen, um auf diese Weise in der Pflanze wertvolle Produkte, z.B. Proteine, zu bilden und nach Überführung der DNA in den Zellkern durch zelleigene Mechanismen zu genetisch neuen Pflanzenrassen zu gelangen. Auch Saccharomyces cerevisiae und Neurospora crassa und deren Protoplasten mit einem Gehalt an durch Lipidvesikel eingebrachter DNA können erfindungsgemäß zur Verfügung gestellt werden.

Es wurde völlig überraschend festgestellt, daß nach dem Einführen von DNA mittels Lipidvesikel in das Cytoplasma von eukaryotischen Zellen, d.h. isolierte Zellen pflanzlichen, tierischen oder menschlichen Ursprungs, eukaryotische Protoplasten oder Zellen von vielzelligen eukaryotischen lebenden Organismen, unmittelbar, nämlich innerhalb weniger Stunden die Produktion der entsprechenden Gen-Produkte festzustellen ist. Ebenso überraschend ist, daß es nach dem Einbringen von DNA-haltigen Lipidvesikeln in vielzellige eukaryotische lebende Organismen im Organismus innerhalb kurzer Zeit zur Produktion der entsprechenden Gen-Produkte kommt. Es war bisher nicht bekannt, daß DNA aus prokaryotischen oder eukaryotischen Quellen in eukaryotischen Systemen sofort und unmittelbar nach dem Einbringen der DNA-haltigen Lipidvesikel, zur Expression gebracht werden konnte. Der Mechanismus der Transkription und Translation von DNA in prokaryotischen und eukaryotischen Systemen ist nocht nicht vollständig aufgeklärt.

Als eukaryotische Zellen kommen für die Erfindung praktisch alle üblichen Zellen und Zell-Linien unterschiedlichen Ursprungs, wie HeLa-Zellen (Mensch), L-Zellen (Maus), Rous Sarcom-Zellen (Huhn), Vero-Zellen (Affe), Nieren-Zellen (Affe, Rind, Schwein) sowie primäre Zellkulturen, die direkt aus beliebigen Organismen, wie z.B. Maus, Ratte, Huhn, Affe, Wachtel, Hamster, Kaninchen, Rind oder Schwein, gewonnen werden. Besonders bevorzugt sind diploide Zellen, die in Gewebekulturen möglichst hohe Generationszahlen erreichen. Ebenso kommen in Frage Zellen und Protoplasten aus z.B. Tabakspflanzen, Getreidepflanzen, Saccharomyces cerevisiae oder Neurospora crassa. Als vielzellige eukaryotische lebende Organismen kommen Pflanzen, Tiere oder Menschen in Frage.

Als DNA-Material aus prokaryotischen oder eukaryotischen Quellen kommen biochemisch gereinigte Gesamt-DNA einer Zelle oder eines Organismus, insbesondere aber hieraus angereicherte Fraktionen (Gen-Fragmente) in Frage. Einsträngige und doppelsträngige DNA, insbesondere spezifische DNA, steht heute nach Replikation in Plasmiden, Phagen oder Viren in beliebigen Mengen zur Verfügung. Vorzugsweise kann Plasmid- und Virus-DNA (Vektoren) verwendet werden, die ihrerseits wiederum bestimmte Spender-DNA aus anderen Organismen beinhalten kann. Dieses Verfahren kommt insbesondere deshalb in Frage, weil hierdurch gewünschte DNA angereichert werden kann. Insbesondere kommen aus den Vektoren herausgeschnittene Gen-Fragmente in Frage, welche codogene DNA-Sequenzen für das gewünschte Gen-Produkt enthalten. Es ist keine notwendige Voraussetzung für die Erfindung, daß die in den Lipidvesikeln eingebrachte DNA nach der Fusion mit dem Zellen oder den Zellen lebender Organismen die Fähigkeit zur Selbstreplikation hat. DNA kann auch mittels reverser Transkription aus RNA hergestellt werden. Es ist ebenfalls möglich, chemisch hergestellte DNA-Sequenzen entweder für sich allein oder nach Kopplung an natürliche oder auf andere Weise gewonnene DNA, z.B. in Plasmiden, zu verwenden.

Die Herstellung der DNA-enthaltenden Lipidvesikel und ihr Einbringen in eukaryotischen Zellen, eukaryotische Protoplasten und vielzellige eukaryotische lebende Organismen erfolgt nach üblichen bekannten Methoden. Insbesondere werden Lipidvesikel nach den von G. Poste, D. Papahadjopoulos und W.J. Vail, a.a.O., sowie R.E. Pagano und J.N.Weinstein, a.a.O., angegebenen Verfahren in die Zellen, Protoplasten oder Organismen eingebracht. Vielzellige eukaryotische lebende Organismen, wie Tiere und Menschen, werden DNA enthaltende Lipidvesikel durch parenterale Injektion, beispielsweise intravenös, intramuskulär, subkutan, intraperitoneal oder durch Injektion in bestimmte Organe, z.B. intratestikulär, appliziert. In Pflanzen können die DNA-haltigen Lipidvesikel in die Gefäße systemisch, z.B. die Leitbündel oder Siebröhren, eingebracht werden.

Mit den Zellen können nach der Fusion mit DNA-haltigen Lipidvesikeln wertvolle primäre Gen-Produkte, wie Antikörper, Enzyme, wie ß-Lactamase, zelluläre, bakterielle oder virale Antigene, wie Tumorantigene, H-Antigene der Salmonellen, Oberflächenantigen des Hepatitis B Virus (HBsAg), Antigene von Tollwut-Virus oder Maul- und Klauenseuche-Virus, hergestellt werden. Es können im Prinzip auch Produkte hergestellt werden, die durch die hintereinander geschaltete Syntheseleistung eines oder mehrerer Enzyme gebildet werden, deren genetische Information durch die Lipidvesikel in die Zellen verbracht wurde. Dasselbe gilt analog für vielzellige Organismen, deren Zellen mit DNA-haltigen Lipidvesikeln zur Fusion gebracht wurden.

Was die Zellen betrifft, so kommen folgende Anwendungen in Frage:
  a) In erster Linie die Möglichkeit, das gewünschte Gen-Produkt sofort zu synthetisieren, ohne daß sich die eingebrachte DNA selbst replizieren muß, und ohne daß die Replikation und Vermehrung dieser Zellen abgewartet werden muß;
  b) infolge der hohen Effizienz des DNA-Transfers durch Lipidvesikel können leicht Zellen isoliert werden, welche die DNA so in ihrem Genom verankert haben, daß die DNA erblich weitergegeben werden kann und zur ständigen Synthese des gewünschten Gen-Produktes führt. Nach dem

0027662

bisherigen Stand der Wissenschaft, d.h. bei den üblichen Transformations-Verfahren, wäre höchstens zu erwarten, daß eine unter etwa 1 Millionen Zellen die gewünschte neue Eigenschaft enthält.

Was vielzellige lebende Organismen betrifft, so können z.B. Tiere zur Bildung von Antikörpern gegen Krankheiten gebracht werden, die durch Parasiten, Bakterien oder Viren verursacht werden. Im Bereich der Humanmedizin können Erbdefekte, wie Galactosämie, Lesch-Nahan-Syndrom, Ahorn-Syrup-Krankheit, Hyperargininaemie, Hämophilie A und B oder Muskeldystrophie behandelt werden. Ebenso können Krankheiten, deren Behandlung die intrazelluläre Synthese von Proteinen im Organismus selbst erforderlich macht, therapiert werden. Durch Fusion von Zellen eines lebenden eukaryotischen Organismus mit DNA-haltigen Lipidvesikeln kann ein Mangel an einem Protein, z.B. einem Peptidhormon oder einem Enzym, beseitigt oder der Schutz gegen virale Infektion (durch Interferonproduktion) erhöht werden.

Das Verfahren zur Herstellung der DNA-haltigen Lipidvesikel besteht im allgemeinen darin, daß bei üblichen pH-Werten, vorzugsweise einem pH-Wert von etwa 7, in wäßrige Lösungen bei Temperaturen, bei denen Lipidvesikel stabil sind, vorzugsweise um 35 bis 37°C, wasserunlösliche polare Lipide (sogenannte amphipathische Lipide), z.B. Phospholipide, entweder allein oder als Lipidgemisch, wie ein Phospholipid, häufig Eilecithin, Cholesterin und ein elektrisch geladenes Amphiphil, wie Stearylamin (positive Ladung) oder Dicetylphosphat (negative Ladung), in unterschiedlichen Molverhältnissen, gewöhnlich im Bereich von 6 : 3 : 1 bis 5 : 5 : 0,5, mit DNA-Material gemischt werden. Sodann werden anschließend, z.B. durch Ultraschall-Behandlung, Lipidvesikel gebildet, die ohne weiteres Zutun das DNA-Material einschließen. Der erhaltenen DNA-haltigen Lipidvesikelsuspension werden erfindungsgemäß eukaryotische Zellen oder Zellkulturen oder eukaryotische Protoplasten als Kulturen zugesetzt. Diese werden nach Routinemethoden angelegt. Vor der Zugabe der DNA-haltigen Lipidvesikelsuspension wird das Kulturmedium teilweise oder ganz entfernt. Nach einer Anzahl von Stunden wird wiederum die Lipidvesikellösung entfernt und neues Kulturmedium zugegeben. Nach etwa 16 bis 24 Stunden kann in den Zellen bzw. Protoplasten eine hinreichende Menge des gewünschten Gen-Produktes nachgewiesen werden.

Als Nachweismethoden für die synthetisierten Gen-Produkte kommen bekannte Verfahren, wie mikrobiologischer Nachweis, Spekralphotometrie, radioimmunologische, virologische und enzymatische Verfahren in Frage. Alle diese Verfahren sind in wissenschaftlichen Standarkwerken veröffentlicht. Als Isolierungs- und Reinigungsmethoden für die Gen-Produkte kommen ebenfalls bekannte Verfahren, wie Chromatographie, Aussalzung, Elektrophorese oder Reaktion mit Antigenen oder Antikörpern, in Frage. Auch diese Verfahren sind in wissenschaftlichen Standardwerken veröffentlicht.

Zur Immunisierung von Tieren und Menschen oder zur Produktion von Antigenen oder Antikörpern können die DNA-haltigen Lipidvesikel in Form üblicher Injektionspräparate nach üblichen Methoden parenteral appliziert werden, z.B. subkutan, intravenös, intramuskulär oder intraperitoneal. Die DNA-haltigen Lipidvesikel können auch unmittelbar in Organe injiziert werden. Es ist klar, daß die Herstellung der DNA-haltigen Lipidvesikelpräparate in diesem Fall unter sterilen Bedingungen erfolgen muß. Injektionspräparate enthalten eine ausreichende Menge der gewünschten DNA-haltigen Lipidvesikel in üblichen Verdünnungsmitteln, z.B. physiologischer Kochsalzlösung. Die DNA-haltigen Lipidvesikelpräparate können ein einziges Mal oder in einem Abstand von Stunden oder Tagen mehrmals injiziert werden. Die verwendete Menge der DNA-haltigen Lipidvesikel, die Tier oder Mensch appliziert wird, ist so bemessen, daß die gewünschte Immunisierung oder die Produktion von Antigenen oder Antikörpern zur Isolierung und Gewinnung ausreichender Mengen erfolgt.

Die Beispiele erläutern die Erfindung.

B e i s p i e l   1

10 µMol Phosphatidylcholin (gereinigt nach W.S. Singleton u. Mitarb., Journal of American Oil Chemist's Society, Bd. 42 (1965), S. 53) und Phosphatidylserin hoher Reinheit aus Rinderhirn im Molverhältnis 9 : 1 werden in 10 ml Chloroform p.a. gelöst. Die erhaltene Lösung wird im Stickstoffstrom bei 36°C zur Trockene eingedampft.

2 µg DNA des ß-Lactamase-Gens (vgl. J.G. Sutcliffe, Proc. Natl. Acad. Sci.   USA , Bd. 75 (1978), S. 3737 bis 3741) werden in 10 ml eines wäßrigen Tris-Histidin-NaCl-Puffers (25 mM Tris-HCl, 2 mM Histidin, 145 mM NaCl; pH 7,4) gelöst. Die erhaltene Lösung wird zu den Lipiden gegeben und das Gemisch wird 30 Minuten bei 35°C unter Stickstoff als Schutzgas mit einem Branson-Ultraschallgerät Typ B-12 derart mit Ultraschall behandelt, daß kein wesentlicher Abbau der DNA erfolgt.

Die erhaltene DNA-haltige Lipidvesikelsuspension wird nun 2 Stunden bei 37°C mit HeLa-Zellen inkubiert. Zu diesem Zweck werden die HeLa-Zellen zunächst mit 20 ml Eagle's Minimal Essential Medium(EMEM) ohne Serum gewaschen. Sodann werden 4 ml der DNA-haltigen Lipidvesikelsuspension (10µMol) zu $10^7$ gewaschenen HeLa-Zellen gegeben.

Nach der Inkubation wird die Lipidvesikelsuspension von den HeLa-Zellen dekantiert. Die Hela-Zellen werden zweimal mit Phosphat-gepufferter Kochsalzlösung (PBS) gewaschen und sodann mit 20 ml EMEM versetzt, das 10 % foetales Kälberserum enthält.

In gleicher Weise werden anstelle von HeLa-Zellen Hühnerembryo-Fibroplasten sowie in einem weiteren Versuch L-Zellen in gleicher Menge verwendet.

Die Genexpression in den HeLa-Zellen, Fibroplasten und L-Zellen wird spektrophotometrisch sowie mikrobiologisch verfolgt. Nach 16 bis 24stündiger Inkubation bei 37°C werden $10^7$ Zellen in üblicher Weise trypsinisiert, sodann bei 270 x g zentrifugiert. Der erhaltene Niederschlag (das "Pellet") wird zum Aufbrechen der Zellen mit Ultraschall behandelt. Aliquots des Zellextrakts werden mit $10^{-4}$ molarer Cephalosporin 87/312 Lösung, d.h. einer Lösung, die 51,6 µg/ml des Cephalosporins enthält (vgl. C.H. O'Callaghan u. Mitarb., Novel Method for Detection of ß-Lactamases by Using a Chromogenic Cephalosporin Substrate, Antimicrobial Agents and Chemotherapy, Bd. 1 (1972), S. 283 bis 288), 30 Minuten bei Raumtemperatur umgesetzt. Die Änderung der Absorptionsspektren der Reaktionsgemische wird mit einem Cary 17 Spektrophotometer aufgezeichnet. Das Maximum bei 386 nm nimmt ab und gleichzeitig erscheint ein neues Maximum bei 482 nm. Dies ist ein Beweis für das Vorliegen von ß-Lactamase im Reaktionsgemisch, die den ß-Lactamring des Cephalosporins geöffnet hat.

Der mikrobiologische Test zur Bestimmung der Anwesenheit von ß-Lactamase, für die das Gen codiert, wird folgendermaßen durchgeführt:
$10^6$ Ampicillin-empfindliche Zellen von E.coli C 600 werden mit 0,5 ml des Zellextrakts in Gegenwart von 30 µg/ml Ampicillin 15 bis 18 Stunden bei 37°C inkubiert. Die Ampicillin-empfindlichen Zellen vermehren sich stark im Medium. Dies ist ein Beweis für die Anwesenheit des Gen-Produkts ß-Lactamase, das den ß-Lactamring des Ampicillins geöffnet und dadurch das Antibiotikum inaktiviert hat.

### Beispiel 2

Beispiel 1 wird wiederholt, jedoch werden anstelle der DNA des ß-Lactamase-Gens 2 µg des DNA-Fragmentes verwendet, das die für HBsAg codierenden DNA-Sequenzen enthält. Die erhaltene DNA-haltige Lipidvesikelsuspension wird zu HeLa-Zellen bzw. zu Hühnerembryofibroplasten gegeben.

Nach mindestens 18stündigem Inkubieren der behandelten Zellen bei 37°C wird das Kulturmedium mittels des Radioimmuntests auf die Gegenwart von HBsAg untersucht. Das Ergebnis des Radioimmuntests zeigt die Gegenwart von HBsAg an.

Das DNA-Fragment, das die für HBsAg codierende DNA enthält, kann nach der von P.Valenzuela u. Mitarb., Nature, Bd. 280 (1979), S. 815 bis 819, beschriebenen Methode, erhalten werden.

Das HBs-Antigen kann durch bekannte biochemische Verfahren, z.B. Sedimentation oder Dichtegradientenzentrifugation, gereinigt werden.

B e i s p i e l   3

Beispiel 2 wird wiederholt, jedoch wird als DNA ein Plasmid verwendet, welches das Gen für das Hepatitis core Antigen (HBcAg) enthält.

Das Ergebnis des Radioimmuntests zeigt die Gegenwart von HBcAg an.

In gleicher Weise können Plasmide verwendet werden, welche Gene für Wachstumshormone, Insulin, Somatostatin (chemisch synthetisierte codogene Sequenzen), Angiotensin II oder Interferon enthalten. Durch den Radioimmuntest oder geeignete biologische Verfahren lassen sich diese Gen-Produkte nach kurzer Zeit nachweisen.

B e i s p i e l   4

Produktion von HBsAg in Kaninchen

Gemäß Beispiel 2 hergestellte Lipidvesikel, die die für HBsAg codierende DNA enthalten, werden in 4 ml physiologischer Kochsalzlösung suspendiert und Kaninchen intravenös in die Ohrvene injiziert. Bis zum 7. Tag werden täglich Serumproben entnommen. Außerdem werden an ausgewählten Tagen Tiere getötet und Leberproben untersucht. Aus den Leberproben wird das Cytoplasma nach bekannten Methoden gewonnen. Im Radioimmuntest läßt sich das HBs-Antigen nachweisen.

Der Hauptzweck dieses Verfahrens ist allgemein betrachtet, darin zu sehen, Tiere durch Produktion von Antigenen und anschließende Immunreaktion, d.h. Bildung von spezifischen Antikörpern, gegen Virus- und andere infektiöse Erkrankungen zu immunisieren.

Patentansprüche

1.   Eukaryotische Zellen mit einem Gehalt an durch Lipidvesikel eingebrachter DNA.

2.   Eukaryotische Zellen nach Anspruch 1, dadurch gekennzeichnet, daß die DNA einsträngig ist.

3.   Eukaryotische Zellen nach Anspruch 1, dadurch gekennzeichnet, daß die DNA doppelsträngig ist.

4.   Eukaryotische Zellen nach Anspruch 1, dadurch gekennzeichnet, daß die DNA prokaryotischen Ursprungs ist.

5.   Eukaryotische Zellen nach Anspruch 1, dadurch gekennzeichnet, daß die DNA eukaryotischen Ursprungs ist.

6. Eukaryotische Zellen nach Anspruch 1, dadurch gekennzeichnet, daß die DNA eine durch reverse Transkription aus RNA hergestellte DNA ist.

7. Eukaryotische Zellen nach Anspruch 1, dadurch gekennzeichnet, daß die DNA eine ganz oder teilweise chemisch erzeugte DNA ist.

8. Eukaryotische Zellen nach Anspruch 1, dadurch gekennzeichnet, daß die DNA eine an ein Plasmid angekoppelte DNA ist.

9. Eukaryotische Zellen nach Anspruch 1, dadurch gekennzeichnet, daß die DNA eine an ein Virus-Genom angekoppelte DNA ist.

10. Eukaryotische Zellen nach einem der Ansprüche 1 bis 9, dadurch gekennzeichnet, daß die eukaryotischen Zellen Zell-Linien sind.

11. Eukaryotische Zellen nach einem der Ansprüche 1 bis 9, dadurch gekennzeichnet, daß die eukaryotischen Zellen primäre Zellkulturen sind.

12. Eukaryotische Zellen nach einem der Ansprüche 1 bis 9, dadurch gekennzeichnet, daß die eukaryotischen Zellen pflanzliche Zellen sind.

13. Eukaryotische Zellen nach einem der Ansprüche 1 bis 9, dadurch gekennzeichnet, daß die eukaryotischen Zellen Protoplasten aus Pflanzen oder Bäckerhefe sind.

14. Eukaryotische Zell-Linien, gekennzeichnet durch einen Gehalt an in die Ausgangszellen durch Lipidvesikel eingebrachter DNA nach einem der Ansprüche 1 bis 9.

15. Vielzellige eukaryotische lebende Organismen mit einem Gehalt an durch Lipidvesikel eingebrachter DNA nach einem der Ansprüche 1 bis 9.

16. Organismen nach Anspruch 15, dadurch gekennzeichnet, daß der Organismus eine Pflanze ist.

17. Organismen nach Anspruch 15, dadurch gekennzeichnet, daß der Organismus ein Tier ist.

18. Organismen nach Anspruch 15, dadurch gekennzeichnet, daß der Organismus ein Warmblüter ist.

19. Organismen nach Anspruch 15, dadurch gekennzeichnet, daß der Organismus ein Säuger ist.

20. Organismen nach Anspruch 19, dadurch gekennzeichnet, daß der Organismus ein Mensch ist.

21. Verfahren zur Herstellung eukaryotischer Zellen mit einem Gehalt an für mindestens ein Gen-Produkt codierender DNA, dadurch gekennzeichnet, daß man DNA-haltige Lipidvesikel gemäß Anspruch 1 bis 9 in die Zellen einbringt.

22. Verfahren zur Herstellung vielzelliger eukaryotischer lebender Organismen mit einem Gehalt an mindestens für ein Gen-Produkt codierender DNA, dadurch gekennzeichnet, daß man DNA-haltige Lipidvesikel gemäß Anspruch 1 bis 9 in den Organismus einbringt.

23. Verfahren nach Anspruch 22, dadurch gekennzeichnet, daß man als Organismus ein Tier verwendet und die DNA-haltigen Lipidvesikel parenteral appliziert.

24. Verfahren nach Anspruch 23, dadurch gekennzeichnet, daß man als Organismus Warmblüter verwendet.

25. Verfahren nach Anspruch 23, dadurch gekennzeichnet, daß man als Organismus Säuger verwendet.

26. Verfahren nach Anspruch 23, dadurch gekennzeichnet, daß man als Organismus Menschen verwendet.

27. Verfahren zur Herstellung von Gen-Produkten, dadurch gekennzeichnet, daß man in die eukaryotischen Zellen oder eukaryotischen Protoplasten DNA-haltige Lipidvesikel gemäß einem der Ansprüche 1 bis 9 einbringt und das primäre oder sekundäre Gen-Produkt isoliert.

28. Verfahren zur Herstellung von Gen-Produkten, dadurch gekennzeichnet, daß man Pflanzen oder Tieren DNA-haltige Lipidvesikel gemäß einem der Ansprüche 1 bis 9 systemisch bzw. parenteral appliziert und aus den Pflanzen oder Tieren das primäre oder sekundäre Gen-Produkt isoliert.

29. Verwendung DNA-haltiger Lipidvesikel gemäß einem der Ansprüche 1 bis 9 zur Herstellung von primären oder sekundären Gen-Produkten in eukaryotischen Zellen, eukaryotischen Protoplasten oder vielzelligen eukaryotischen lebenden Organismen.

30. Verwendung DNA-haltiger Lipidvesikel gemäß einem der Ansprüche 1 bis 9 zur Immunisierung von Warmblütern.

31. Verwendung DNA-haltiger Lipidvesikel gemäß einem der Ansprüche 1 bis 9, bei denen die DNA für ein Gen-Produkt codiert, das eukaryotischen Zellen, eukaryotischen Protoplasten oder vielzelligen eukaryotischen lebenden Organismen fehlt, zur Behebung der entsprechenden genetisch bedingten Defekte.

Europäisches
Patentamt

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| DX | NUCLEIC ACIDS RESEARCH, Band 6, Nr. 12, 1979, P.F. LURQUIN: "Entrapment of plasmid DNA by liposomes and their interactions with plant protoplasts", Seiten 3773-3784 <br><br> * Seite 3373, letzter Absatz; Seiten 3782-3783; Ansprüche * | 1,3,4 8,12, 13,21 |
| DX | PROC. NATL. ACAD. SCI. USA, Band 75, Nr. 3, März 1978, A.B. MUKHERJEE et al.: "Entrapment of metaphase chromosomes into phospholipid vesicles (lipochromosomes): Carrier potential in gene transfer", Seiten 1361-1365 <br><br> * Seite 1361; Zusammenfassung; Tabelle 2; Seiten 1364-1365; Diskussion * | 1,3,5 10,14 21,27 29,31 |
| D | NUCLEIC ACIDS RESEARCH, Band 6, Nr. 8, 1979, G.J. DIMITRIADIS: "Entrapment of plasmid DNA in liposomes", Seiten 2697-2705 <br><br> * Seite 2702, Zeilen 7-12; Tabelle 2; Seite 2704, Zeilen 8-18 * | |
| D | PROC. NATL. ACAD. SCI. USA, Band 76, Nr. 7, Juli 1979, R.T. FRALEY et al.: "Entrapment of a bacterial plasmid in phospholipid vesicles: Potential for gene transfer", Seiten 3348-3352 <br> ./. | 1,8, 21,27, 29 |

**KLASSIFIKATION DER ANMELDUNG (Int Cl³)**

```
C 12 N  15/00
A 61 K  31/70
        39/00
         9/50
A 01 K  67/00
A 01 H   1/00
C 12 N   5/00
         1/16
```

**RECHERCHIERTE SACHGEBIETE (Int Cl³)**

```
C 12 N  15/00
A 61 K   9/50
        31/70
        39/00
        67/00
A 01 H   1/00
```

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 16-01-1981 | DESCAMPS |

EPA form 1503.1  06.78

| | EINSCHLÄGIGE DOKUMENTE | | KLASSIFIKATION DER ANMELDUNG (Int Cl.³) |
|---|---|---|---|
| Kategorie | Kennzeichnung des Dokuments mit Angabe, soweit erforderlich, der Maßgeblichen Teile | betrifft Anspruch | |
| | * Seite 3338, Zusammenfassung und Absatz 1; Seite 3352, Spalte 1, letzter Absatz bis Spalte 2 ende *<br><br>-- | | |
| | CHEMICAL ABSTRACTS, Band 91, Nr. 7, 13. August 1979, Seite 242, Zusammenfassung Nr. 51516j, Columbus, Ohio, US, R.J. MANNINO et al.: "Encapsulation of high molecular weight DNA in large unilamellar phospholipid vesicles. Dependence on the size of the DNA"<br><br>& FEBS LETT. 1979, 101(2), 229-32<br><br>-- | 1 | RECHERCHIERTE SACHGEBIETE (Int Cl.³) |
| PX | CHEMICAL ABSTRACTS, Band 93, Nr. 13, 29. September 1980, Seite 236, Zusammenfassung Nr. 127581r, Columbus, Ohio, US, WONG TAI-KIN et al.: "Appearance of $\lambda$-lactamase activity in animal cells upon liposome-mediated gene transfer"<br><br>& GENE, 1980, 10(2), 87-94<br><br>-- | 1,3,4, 8,10, 14,21, 27,29, 31 | |
| PX | CHEMICAL ABSTRACTS, Band 92, Nr. 11, 17. März 1980, Seite 317, Zusammenfassung Nr. 91076t, Columbus, Ohio, US, P.F. LURQUIN: "Transfer of plasmid DNA to plant protoplasts. II Use of DNA sequestered in liposomes"<br><br>& ARCH. INT. PHYSIOL. BIOCHIM., 1979, 87(4), 825-6<br><br>-- | 1,3,4, 8,12, 13,21 | |
| A | CHEMICAL ABSTRACTS, Band 91, Nr. ./. | | |

EPA Form 1503.2   06.78

0027662

Nummer der Anmeldung

EP 80 10 6503

Europäisches Patentamt

**EUROPÄISCHER RECHERCHENBERICHT**

| EINSCHLÄGIGE DOKUMENTE | | | KLASSIFIKATION DER ANMELDUNG (Int Cl³) |
|---|---|---|---|
| Kategorie | Kennzeichnung des Dokuments mit Angabe, soweit erforderlich, der maßgeblichen Teile | betrifft Anspruch | |
| | 21, 19. November 1979, Seite 321, Zusammenfassung Nr. 171201z, Columbus, Ohio, US, D. PAPAHADJOPOULOS et al.: "The use of lipid vesicles for introducing macromolecules into cells" & PROTEIN TURNOVER LYSOSOME FUNCT. (Proc. Symp.) 1977, 543-60 | | |
| A | CHEMICAL ABSTRACTS, Band 90, Nr. 23, 4. Juni 1979, Seite 339, Zusammenfassung Nr. 183224b, B. MATTHEWS et al.: "Liposome-mediated transfer of bacterial RNA into carrot protoplasts" & PLANTA, 1979, 145(1), 37-44 | | RECHERCHIERTE SACHGEBIETE (Int Cl³) |
| A | NATURE, Band 274, 31. August '78, M.J. OSTRO et al.: "Evidence for translation of rabbit globin mRNA after liposome-mediated insertion into a human cell line", Seiten 921-923 | | |
| DA | NATURE, Band 274, 31. August '78, G.J. DIMITRIADIS: "Translation of rabbit globin mRNA introduced by liposomes into mouse lymphocytes" Seiten 923-924 | | |
| DA | CELL, Band 17, Mai 1979, T. WILSON et al.: "The introduction of poliovirus RNA into cells via lipid vesicles (Liposomes)" | | |
| A | EP - A - 0 004 223 (D.P. PAPAHADJOPOULOS) | | |

EPA Form 1503.2  06.78